Europäisches Patentamt

(19) European Patent Office

Office européen des brevets

(11) Publication number : **0 335 140 B1**

(12) **EUROPEAN PATENT SPECIFICATION**

(45) Date of publication of patent specification :
04.03.92 Bulletin 92/10

(51) Int. Cl.⁵ : **A61K 31/59, A61K 33/10**

(21) Application number : **89104086.7**

(22) Date of filing : **08.03.89**

(54) Compositions for the treatment of osteoporosis in humans.

(30) Priority : **23.03.88 US 172699**

(43) Date of publication of application :
04.10.89 Bulletin 89/40

(45) Publication of the grant of the patent :
04.03.92 Bulletin 92/10

(84) Designated Contracting States :
**AT BE CH DE ES FR GB GR IT LI LU NL SE**

(56) References cited :
**DIALOG 5279504, Embase no. 83030433; J.
REEVE et al.: "Long-term treatment of
osteoporosis with 24,25 dihydroxycholecal-
ciferol", & ACTA ENDOCRINOL. 1982, 101/4
(636-640)**

(73) Proprietor : **Teva Pharmaceutical Industries
Limited
Har Hotsvim behind Sanhedria Ha'Murhevet
Jerusalem (IL)**
Proprietor : **YEDA RESEARCH AND
DEVELOPMENT COMPANY, LTD.
Weizmann Institute of Science P.O. Box 26
Rehovot (IL)**

(72) Inventor : **Edelstein, Samuel
23 Miller Street
Rehovot 76 284 (IL)**
Inventor : **Ladkani, David
29/19 Gwatemala Street
Kiryat Yovel Jerusalem 96 704 (IL)**
Inventor : **Kadosh, Shaul
Ma'alot Dafna 126/26
Jerusalem 97 763 (IL)**
Inventor : **Shalita, Beni
44 Ramat Hagolan Street
Ramat Eshcol Jerusalem 97 704 (IL)**
Inventor : **Mazor, Ze'ev
Mevo Harari
Jerusalem 97 886 (IL)**
Inventor : **Weiner, Ben Zion
306/16 Hapartizanim Street
Hagiva Hatzarfatit Jerusalem 97 888 (IL)**
Inventor : **Menczel, Jacob
30 Tshernekofsky Street
Jerusalem 92 585 (IL)**

(74) Representative : **Brown, John David et al
FORRESTER & BOEHMERT
Widenmayerstrasse 4/I
W-8000 München 22 (DE)**

## Description

This invention relates to a composition for the treatment of osteoporosis in humans, in particular post-menopausal osteoporosis (hereinafter "PMO") in women in the post-menopausal state. More particularly, the invention provides a composition for the alleviation and prevention of osteoporosis in humans, which composition comprises calcium and 24,25-dihydroxycholecalciferol (24,25-dihydroxy-vitamin $D_3$, hereinafter "24,25 $(OH)_2D_3$").

Osteoporosis is the commonest of the metabolic bone diseases and is characterized by a reduction in the bone mass per unit volume to a level below that required for the adequate mechanical support function of the bone. The decrease in bone mass in osteoporosis indicates that the rate of bone resorption exceeds that of bone formation. Osteoporosis increases with age and is of higher incidence in women than in men. It is well known that women in the post-menopausal state often suffer from a marked loss of bone mass and as a consequence are susceptible to spontaneous fractures of the vertebrae and of the long bones. PMO is often accompanied by bone pain, deformity of the spine, decreased physical activity and trauma and presents a worldwide major medical problem. This disease is characterised by a diminished ability to absorb calcium, a negative calcium balance and decreased levels of sex hormones, especially estrogen. The bone mass in PMO can be assayed by means of lateral lumbar spine radiographs, by single and dual photon absorptionmetry and the vertebrae microfracturing can be diagnosed by X-ray evidence.

Various methods have been proposed and some are still in practice, for treating osteoporosis and particularly PMO, but none of these methods has proven to be completely satisfactory. It is especially noteworthy that calcium supplementation by itself does not prevent or cure the disease, having only a minor effect on the loss of cortical bone, but no effect on the trabecular bone (Riis, B. et al. N. Engl. J. Med., 316, 173 (1987)). Administration of sex hormones, especially estrogen, has been reported to be effective in preventing the loss of bone mass in PMO, but this method involves a serious risk of possible carcinogenicity (mainly endometrial carcinoma), particularly when relatively high dosages of hormones are administered. The recently introduced estrogen/progestrogen treatment has somewhat reduced this risk which, however, still presents a serious problem.

Some favourable results have been reported for another treatment of osteroporosis, namely the administration of large doses of calcium together with fluorides and vitamin D. However, this treatment induces structurally unsound bones, the so-called "woven bone" and additionally has some other undesired side effects, such as increased incidence of fractures and gastro-intestinal reactions.

The calcium regulating horome calcitonin has often been prescribed in the treatment of PMO patients who were unsuitable candidates for estrogen replacement therapy. While offering marked relief of pain in PMO patients, the lasting beneficial effect of this therapy is doubtful owing to the development of resistance (Lancet (Editorial), P. 833, Oct. 10, 1987). In addition, the use of this treatment is limited because of the high cost of calcitonin.

The therapeutic application of various vitamin D metabolites for the treatment of osteoporosis has recently received much attention. Thus, De Luca, U.S. Patent No. 4,225,596 proposed the administration of 1,25-dihydroxycholecalciferol (hereinafter "1,25$(OH)_2D_3$"), the active metabolite of vitamin $D_3$, or alternatively 1α-hydroxycholecalciferol (hereinafter "1α$(OH)D_3$") which is metabolised by the liver to 1,25$(OH)_2D_3$. These metabolites are reported to have beneficial effects on the absorption of calcium and on the calcium balance in the bone. However, the treatment with 1,25$(OH)_2D_3$ or 1α$(OH)D_3$ is liable to produce serious side effects, such as hypercalcemia, hypercalcuria and an unfavourable effect on the urinary calcium/creatinine ratio which serves as an indication of the renal function. In addition, the beneficial effects on the mineralisation of the bone matrix are incomplete and unsatisfactory. High doses of these metabolites may even cause damage to the bone owing to increased bone resorption.

Another metabolite of vitamin $D_3$, 24,25-dihydroxycholecalciferol (hereinafter "24,25$(OH)_2D_3$") has recently been indicated in the literature as possibly having beneficial effects in the treatment of osteoporosis and as improving some parameters of bone mineralization (cf. Kanis et al., Brit. Med. J. 1382, (1978); Garabedian et al., Clin. Orthomedics & Rel. Res., 135, 241 (1978); Lawoyin et al., J. Clin. Endocrinol Metabol., 50(3), 593 (1980); Reeve et al., Acta Endocrinol 101, 636 (1982) inter alia). However, all these publications merely speculated on the possible favourable effects of 24,25$(OH)_2D_3$ in osteoporosis but did not clearly indicate any method or dosage units to be used in the therapy of human osteoporosis or PMO. The above applies also to U.S. Patent No. 4,590,184 (Y. Maeda et al.) which claims a method for the treatment of osteoporosis by administration of 24,25$(OH)_2D_3$. In this patent there are described only tests performed with ovariectomized rats as a model of PMO in humans, but the possible beneficial effects on the bone mass rate of fracture and clinical symptoms in human patients are not reported. Furthermore, the daily dosage indicated in this patent is "0.01 to 100,000 μg, preferably 0.5 to 10,000 μg" and it is clear that such excessively wide ranges provide the medical practitioner with no guidance whatsoever how to practice the method for the treatment of osteoporosis which is claimed in said U.S. Patent 4,590,184.

In accordance with one aspect of the present invention there is provided a method for the alleviation or prevention of osteoporosis in a human patient, which comprises a combination of a dosage unit of from 10 to 40 $\mu$g (mcg) of 24,25 $(OH_2)D_3$ and from 250 to 750 mg per day of calcium in combination with from 1g to 1.5g per day of calcium in the form of a conventional dietary calcium supplement. The preferred daily dosages are 40 $\mu$g (mcg) of 24,25$(OH)_2D_3$ and 1g of calcium.

The present invention is based on the surprising discovery that remarkable improvements can be achieved in osteoporosis patients, particularly PMO patients, by the combined administration of 24,25$(OH)_2D_3$ and conventional amounts of calcium supplement, only when the 24,25$(OH)_2D_3$ is administered at certain daily dosages which are considerably higher than those hitherto recommended and which, on the basis of the available prior art, would have been considered to be dangerously excessive and liable to cause serious untoward side effects, such as hypercalcemia, hypercalcuria and an unfavourable urinary calcium/creatinine ratio.

On the basis of long-term controlled and randomised clinical studies with 122 PMO patients, the favourable daily dosage of 24,25$(OH)_2D_3$ was determined to be in the range of 20 to 80 $\mu$g (mcg) (mcg/d), preferably 40 $\mu$g (mcg)/d, and the daily dosage of the dietary calcium supplement was determined to be such as to contain from 1.0 to 1.5g, preferably 1.0g, of calcium. At these dosage ranges remarkable improvements were observed in the bone mineral content (BMC) of the patients and the treatment proved to be completely safe, i.e. free of any toxicity and side effects, such as hypercalcemia or hypercalcuria, while retaining a favourable urinary calcium/creatinine ratio.

The preferred method of treatment of osteoporosis comprises the administration to a patient of 40 $\mu$g (mcg) per day of 24,25$(OH)_2D_3$ in two dosage units of 20 $\mu$g (mcg) each, one in the morning and one in the evening, in addition to one gram of ionic calcium in the form of a conventional calcium supplement per day, preferably in the form of two tablets per day each containing 500 mg of ionic calcium.

The daily or twice-daily doses of 24,25$(OH)_2D_3$ are preferably administered orally, e.g. in the form of capsules, soft gelatine capsules, tablets, granules or solutions. However, various other manners of administration are contemplated, such as in suppositories, interperitoneally or transdermally.

The preferred manner of administration of 24,25$(OH)_2D_3$ in accordance with the invention is by means of soft gelatine capsules containing inert carriers or excipients, such as arachis oil or other suitable oils, triglycerides of fatty acids, glycerol, propylene glycol or other suitable inert solvents, optionally together with stabilizers and/or preservatives.

An alternative preferred manner of administration of the 24,25$(OH)_2D_3$ and the ionic calcium in accordance with the invention is in the form of a pharmaceutical combination comprising both the aforesaid active ingredients, together with any of the above mentioned excipients or carriers and/or additives. For example, such a pharmaceutical combination would comprise 20 $\mu$g (mcg) of 24,25$(OH)_2D_3$ and an amount of a conventional dietary calcium supplement containing 500 mg of ionic calcium. As stated above, such pharmaceutical compositions form an aspect of the present invention.

Dietary calcium supplements suitable for use in the method and compositions according to the present invention are those conventionally used for supplementing the normal diet with calcium, mostly any salt of calcium with an anion or anions which are non-toxic to the human body. Examples of such non-toxic calcium salts include the carbonate, phosphate, chloride, lactate, citrate and gluconate.

The clinical studies will now be described in detail and their results illustrated in the accompanying drawings, in which:

Fig. 1 is a graphical representation of the percent of change in bone mineral content (BMC) in a group of patients treated with 0.5 $\mu$g (mcg)/d of 1$\alpha$(OH)$D_3$ and 1g/d of calcium supplement, as compared to a control group of patients treated with 1g/d of calcium supplement only;

Fig. 2 is a graphical representation of the percent of change in BMC in a group of patients treated with 4-10 $\mu$g (mcg)/d of 24,25$(OH)_2D_3$ and 1g/d of calcium supplement, as compared to a control group treated with 1g/d of calcium supplement only;

Fig. 3 is a graphical representation of the percent of change in BMC in a group of patients treated with 40 $\mu$g (mcg)/d of 24,24$(OH)_2D_3$ and 1g/d of calcium supplement, as compared to a control group treated with 1g/d of calcium supplement only;

Fig. 4 is a comparative graphical representation of the percent of change in BMC in the group of patients treated with 0.5 $\mu$g (mcg)/d of 1$\alpha$(OH)$D_3$ and 1g/d of a calcium supplement, versus the group treated with 40 $\mu$g (mcg)/d of 24,25$(OH)_2D_3$ and 1g/d of calcium supplement;

Fig. 5 is a diagram showing the percent of hypercalcemic episodes (more than 10.8 mg/100 ml) in patients of the two groups mentioned in connection with Fig. 4 above and of the control group;

Fig. 6 is a graphical representation of the total urine calcium/24 hrs. in the two groups of patients mentioned in connection with Fig. 1 above;

Fig. 7 is a graphical representation of the total urine calcium/24 hrs. in the two groups of patients mentioned in connection with Fig. 3 above;

Fig. 8 is a comparative graphicl representation of the total urine calcium/24 hrs. in the two groups of patients mentioned in connection with Fig. 4 above;

Fig. 9 is a graphical representation of the urinary calcium/creatinine ratio in the two groups of patients mentioned in connection with Fig. 1 above;

Fig. 10 is a graphicl representation of the urinary calcium/creatinine ratio in the two groups of patients mentioned in connection with Fig. 3 above; and

Fig. 11 is a comparative graphical representation of the urinary calcium/creatinine ratio in the two groups of patents mentioned in connection with Fig. 4 above.

## CLINICAL STUDIES

The studies were performed during the period from January 1, 1982 to December 31, 1986, on a total of 122 female PMO patients ranging in age from 44 to 88 years.

The patients were randomly allocated into the following four groups and all patients in all groups were administered 1g of ionic calcium per day orally in the form of Calcium Sandoz Forte, containing calcium lacticoglyconicum 2.94g and calcium carbonicum 0.3g, equivalent to 500 mg ionic calcium (two tablets daily):

1. One group of 25 patients received 1g of calcium per day together with a daily dosage of $24,25(OH)_2D_3$ as follows: During the first year, 4 mcg daily (2 x 2 $\mu$g (mcg)/d). From the beginning of the second year the dose was raised to 10 $\mu$g (mcg)/d (2 x 5 mcg/d). An additional group of six patients received from the start of the treatment 10 $\mu$g (mcg)/d (2 x 5 mcg/d) of $24,25(OH)_2D_3$.

2. A second group of 25 patients received (starting in 1984) 1g of calcium per day together with 40 $\mu$g (mcg)/d of $24,25(OH)_2D_3$ (2 x 10 $\mu$g (mcg)/d).

3. A comparison group of 24 patients received (starting in 1984) 1g of calcium per day together with 0.5 $\mu$g (mcg)/d (2 x 0.25 $\mu$g (mcg)/d) of $1\alpha(OH)D_3$.

4. A control group consisted of 42 patients receiving 1g of calcium per day together with a placebo.

Of the 122 patients who entered the study, the groups treated with $24,25(OH)_2D_3$ consisted of 56 patients representing a total of 1772 "patient treatment months" (equal to about 147 total "patient treatment years").

The patients' monthly follow-up included medical interviews to specify any complaints, side effects or other abnormalities, as well as biochemicl tests of blood and urine regarding kidney function and mineral metabolism. The effects of the treatment on the skeleton were evaluated by lateral lumbar spine radiographs and the metacarpal cortical index of the non-dominant hand, performed twice a year. Additionally, bone density and bone mineral content (BMC) of the distal radius were evaluated at a frequency of 3 to 6 months. These tests are described hereinafter in more detail.

## METHODS OF ASSAY OF OSTEOPOROSIS

The patients were selected for these studies on the basis of their diagnosis for osteoporosis made on lateral X-ray radiographs of the lumbar spine taken under standard conditions. Therefore, the main method of estimation of bone mass and possible changes thereof was chosen to be the X-ray radiographs. X-rays were taken on the same machine and by the same technician and films were developed in an automatic processing unit. In addition a PA (Posterior-Anterior) X-ray of the non-dominant hand was taken for evaluation of the cortical index of the third metacarpal, which in previous studies was shown to have a high correlation with the qualitative estimation of the lumbar vertebrae.

## BONE SCANNING

A photon-absorptionmeter (The Norland Digital Bone Densitometer) was used to assess the mineral content of mainly cancellous bone structures, such as the distal end of the radius, using a standard site for scanning at the base of the radial styloid 3cm proximal to the tip.

## BIOCHEMICAL STUDIES

Blood samples were taken monthly. Levels of calcium, phosphate, magnesium and creatinine were measured both in serum and urine as well as serum BUN (Blood Urea Nitrogen) and alkaline phosphatase levels. Vitamin $D_3$ metabolites ($25(OH)D_3$; $24,25(OH)_2D_3$; and $1,25(OH)_2D_3$) were determined throughout the study.

## BIOCHEMICAL RESULTS

No significant change in serum calcium levels was detected in any of the above mentioned groups of patients during the whole period of treatment. Urinary calcium remained within the normal range in patients who were treated with calcium supplement alone or in combination with $24,25(OH)_2D_3$. In the groups treated with calcium supplement in combination with $1\alpha(OH)D_3$, urinary calcium increased substantially, reaching a level of over 250 mg/d in 43% of the patients (this phenomenon is well documented in the literature). Despite this hyper-calcuria, serum creatinine, BUN and creatinine clearance were not affected. It should be noted that none of the patients developed a kidney stone during the study period. No changes in any other biochemical parameters were observed during the whole period of treatment in any of the treatment groups.

## EFFICACY OF TREATMENT

The efficacy of the treatment on the bone mass in the various groups of patients was best determined by means of the assay of bone mineral content (B.M.C.) and its changes (in percent) during the treatment. The folowing results were obtained in the above mentioned long-term controlled studies:

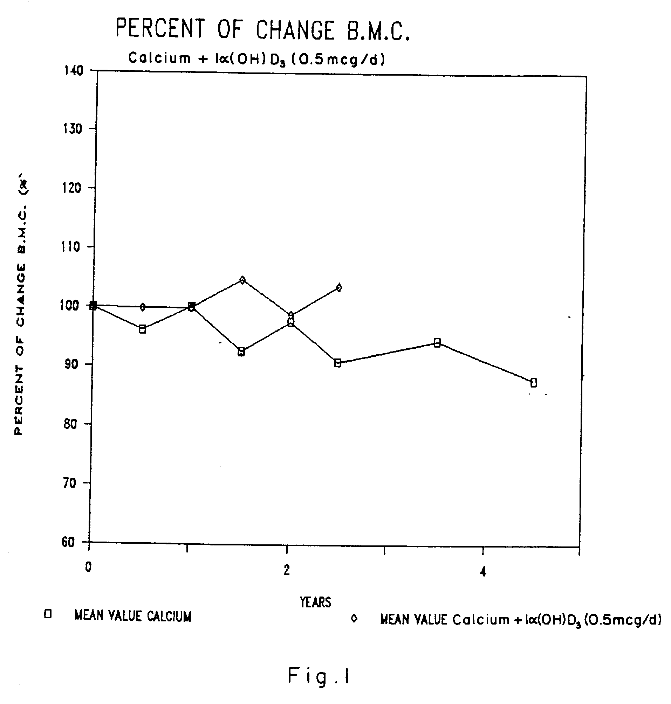

1) The group (3) above which was treated with $1\alpha(OH)D_3$ and the calcium supplement, showed an improved percentage change in the B.M.C. of the patients as compared to the control group patients who received the calcium supplement alone (see Fig. 1).

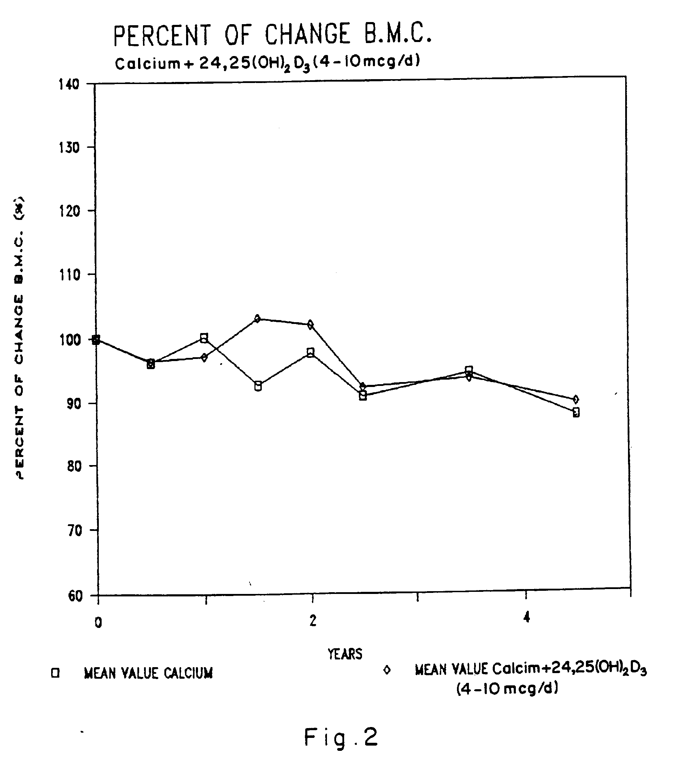

2) The group (1) which was treated with the low daily dosage of $24,25(OH)_2D_3$ at 4-10 μg (mcg)/d plus the calcium supplement, showed a percentage change in the B.M.C. almost equal to that of the control group and lower than that of group (3) discussed above (see Fig. 2).

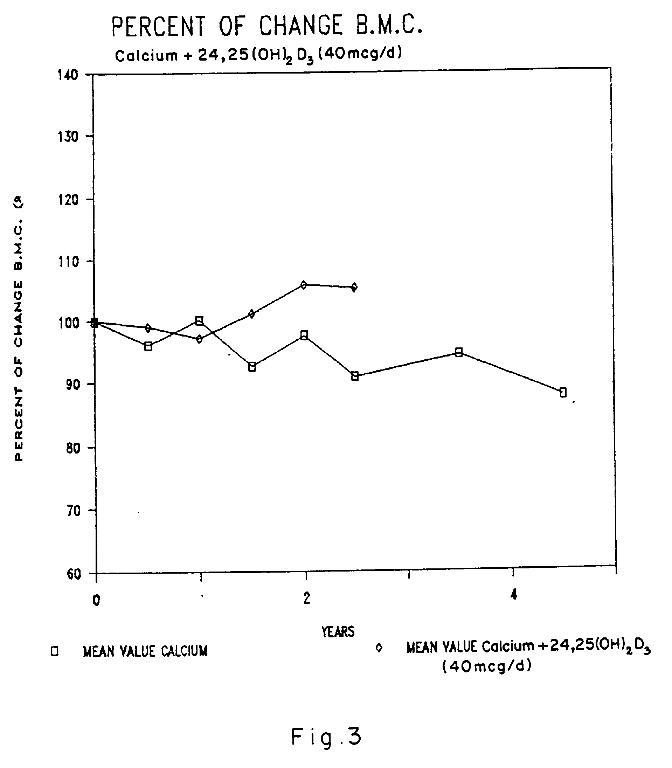

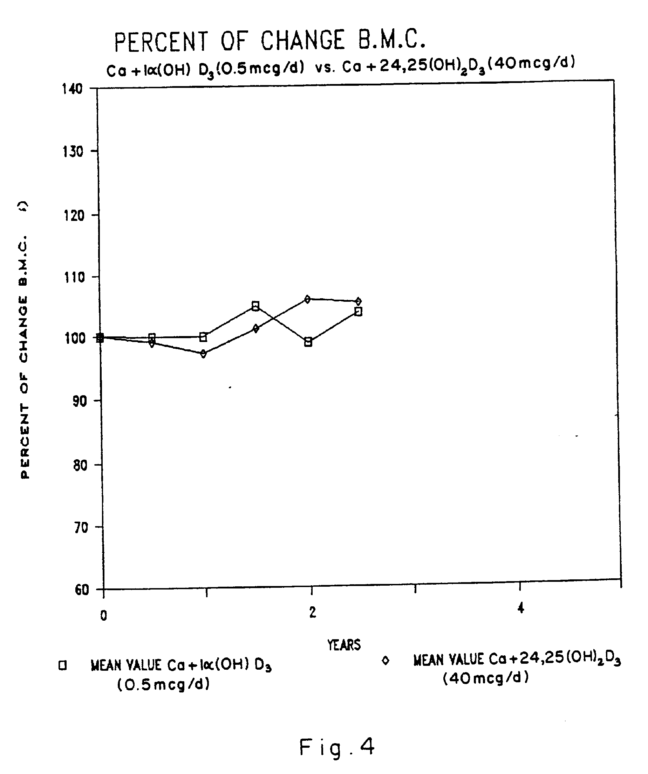

3) Group (2) which was treated with the high daily dosage of $24,25(OH)_2D_3$, i.e. 40 μg (mcg)/d plus the calcium supplement, showed an improved percentage change in the B.M.C. patients which was at least equal to that observed in group (3) discussed above (see Figs. 3 and 4).

The difference in the bone fracture rates of the various treatment groups could not be determined statistically, since the total "patient treatment years" in each of the groups were insufficient for this purpose. However, from the improvement in bone mass of the group (2) which was treated with 40 μg (mcg)/d of $24,25(OH)_2D_3$, as indicated by the higher percentage change in B.M.C., it may be deduced that such a treatment would, in the long-term, achieve a considerable reduction in the bone fracture rate. A similar deduction may be made also in respect of the symptoms of lower-back pains, for which no substantial improvement could be statistically determined in the group (2) (40 μg (mcg)/d of $24,25(OH)_2D_3$) in comparison with the group (3) ($1\alpha(OH)D_3$) or with the control group, owing to the comparatively short periods of treatment.

It should further be noted that no change in the cortical index was observed during the entire treatment in group (2), i.e. the patients treated with 40 μg (mcg)/d of $24,25(OH)_2D_3$ plus the calcium supplement.

## SAFETY OF THE TREATMENT

The various methods of treatment were evaluated as regards the safety by means of the three following main criteria:

1) The number of hypercalcemic episodes. (Hypercalcemia is defined as a serum calcium level higher than 10.8 mg %);

2) Hypercalcuria as determined by the total urine calcium content (mg/24 hrs); and

3) Urinary calcium/creatinine ratio.

The following results were observed:

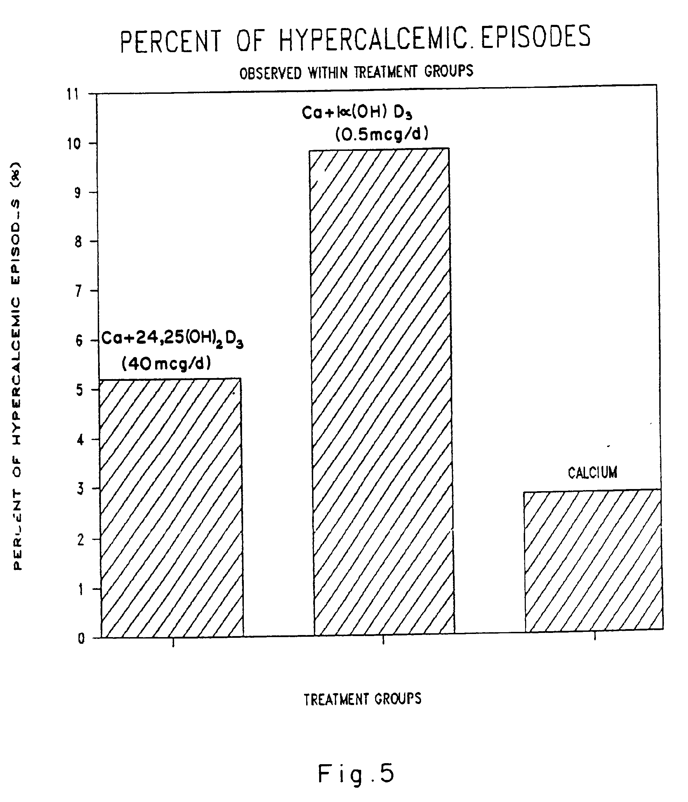

1. The treatment group (3) receiving $1\alpha(OH)D_3$ exhibited the highest percent of hypercalcemic episodes, whereas group (2) treated with the high dosage of $24,25(OH)_{23}$ had about half as much hypercalcemic episodes, only somewhat higher than that of the control group treated with the calcium supplement alone (see Fig. 5).

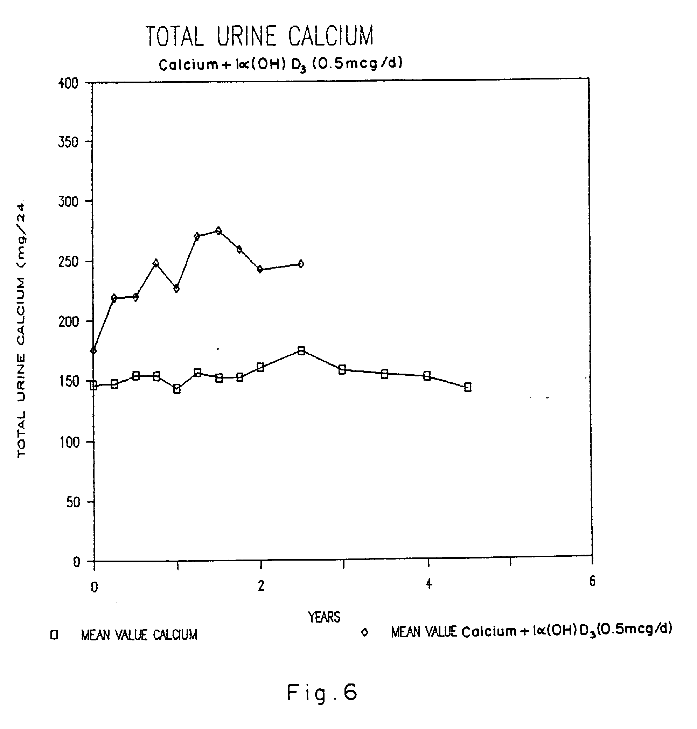

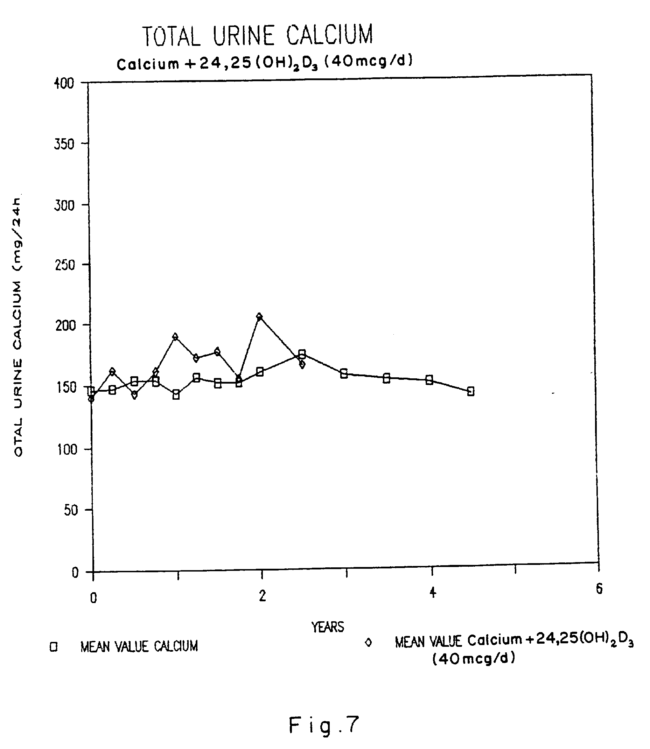

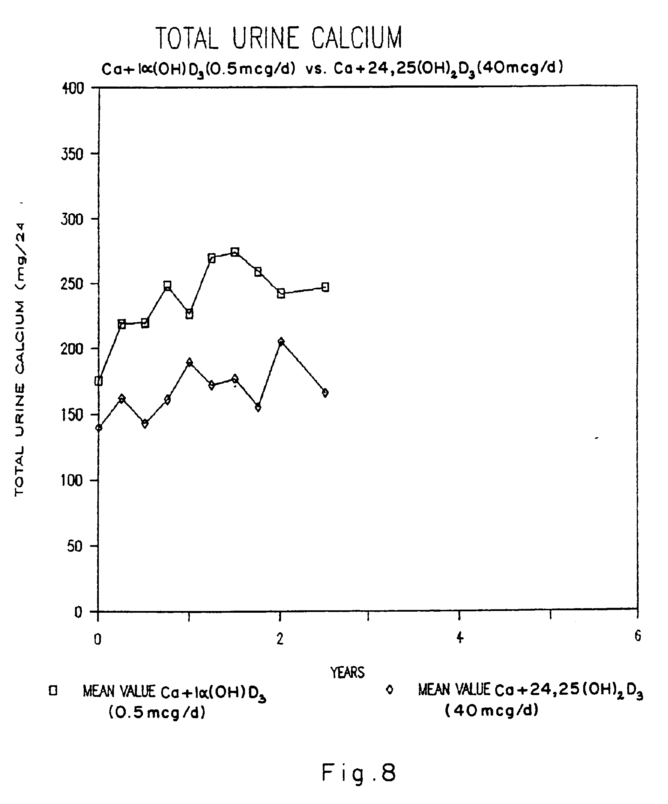

2. Treatment group (3) receiving $1\alpha(OH)D_3$ showed a significantly higher average excretion of calcium in the urine as compared with that of the group (2) treated with the high dosage $24,25(OH)_2D_3$ (40 μg (mcg)/d). This finding, which is illustrated in Figs. 6, 7 and 8, shows that treatment with $24,25(OH)_2D_3$ reduces the danger of hypercalcuria as compared to the treatment with $1\alpha(OH)D_3$.

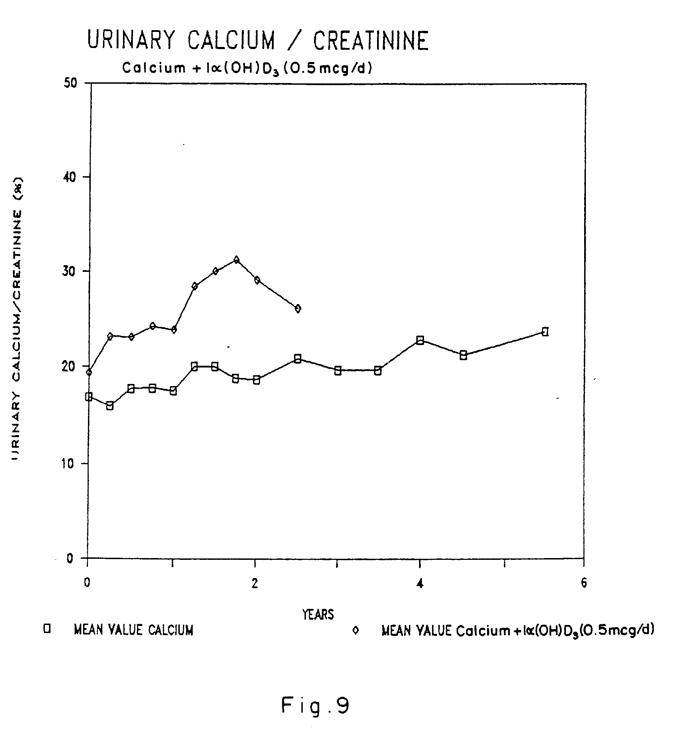

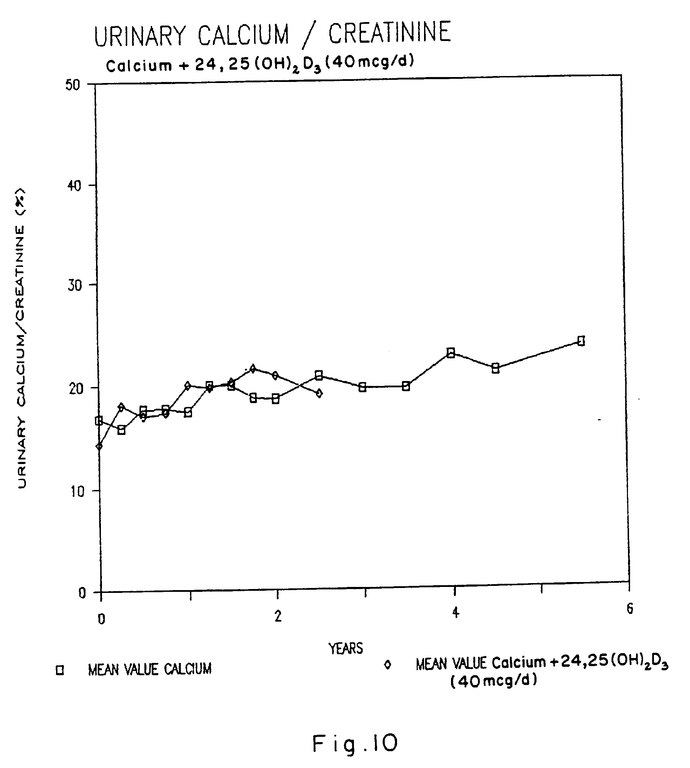

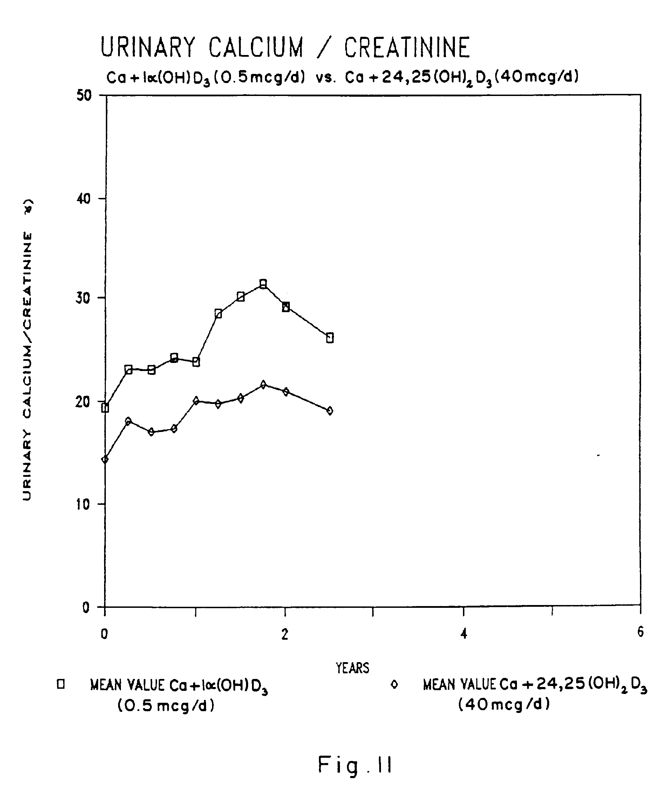

3. As regards the urinary calcium/creatinine ratio it was also found that the levels of this ratio were higher in the group (3) treated with $1\alpha(OH)D_3$ than in group (2) which was treated with the higher dosage of 40 μg (mcg)/d of $24,25(OH)_2D_3$ (see Figs. 9, 10 and 11).

OTHER SIDE EFFECTS

The studies also showed that daily dosages of 4, 10 and 40 µg (mcg) of $24,25(OH)_2D_3$ administered together with 1 g/d of calcium did not induce significant side effects, the exception being gastrointestinal complaints. The high prevalence of gastrointestinal symptoms, especially constipation, can be attributed to the advanced age of many of the tested patients as well as to the known effect of calcium supplementation. The latter conclusion is supported by the fact that the prevalence of gastrointestinal symptoms in the patients treated with $24,25(OH)_2D_3$ was not higher than in the control group of patients who received the calcium supplement alone.

Thus, it can be concluded that the method of treatment according to the present invention is practically free of any undesired side effects.

PHARMACEUTICAL COMPOSITIONS

The preparation of pharmaceutical compositions suitable for use in the method according to the invention is illustrated in the following non-limiting examples:

EXAMPLE 1

Soft Gelatine Capsules of 5.0 µg (mcg) of 24,25 $(OH)_2D_3$

Each 5.0 mcg capcule contains:

| | |
|---|---|
| 24R,25-Dihydroxycholecalciferol | 5.500 µg (mcg)* |
| Citric acid, anhydrous | 0.015 mg |
| Propyl gallate | 0.020 mg |
| Vitamin E (dl-alpha Tocopherol) | 0.020 mg |
| Alcohol, dehydrated | 1.145 mg |
| Peanut oil (Arachis oil) | 98.800 mg |
| Total fill weight per capsule: | 100.0 mg |

*Includes 10% excess.

EXAMPLE 2

Soft Gelatine Capsules of 10.0 μg (mcg) of 24,25 (OH)$_2$D$_3$

```
24R,25-Dihydroxycholecalciferol          11.000 µg (mcg)*

    Citric acid, anhydrous                0.015 mg

    Propyl gallate                        0.020 mg

    Vitamin E (dl-alpha Tocopherol)       0.020 mg

    Alcohol, dehydrated                   1.145 mg

    Peanut oil (Arachis oil)             98.800 mg

    Total fill weight per capsule: 100.0 mg

*Includes 10% excess.
```

EXAMPLE 3

Soft Gelatine Capsules of 20 μg (mcg) of 24,25 (OH)$_2$D$_3$

```
    24R,25-Dihydroxycholecalciferol       22.000 µg (mcg)*

    Citric acid, anhydrous                0.015 mg

    Propyl gallate                        0.020 mg

    Vitamin E (dl-alpha Tocopherol)       0.020 mg

    Alcohol, dehydrated                   1.145 mg

    Peanut oil (Arachis oil)             98.800 mg

    Total fill weight per capsule: 100.0 mg

*Includes 10% excess.
```

EXAMPLE 4

Compressed Tablet

5-40 μg (mcg) of 24,25(OH)$_2$D$_3$ are adsorbed onto a suitable substrate (e.g. silica or cellulosic material) and progressively diluted (triturated) and dispersed in a calcium carbonate granulate containing the equivalent of 250 mg to 500 mg of calcium. The granulate is compressed into a tablet.

EXAMPLE 5

Film-Coated Tablet

5-40 μg (mcg) of 24,25(OH)$_2$D$_3$ are dispersed or dissolved in an organic solvent or aqueous mix, admixed with a pharmaceutically acceptable polymer and spray coated onto a tablet core containing calcium carbonate (in an amount equivalent to 250 mg to 500 mg of calcium). Additional protective coating may be added.

7

EXAMPLE 6

Press-Coated Tablet

(a) 5-40 µg (mcg) of 24,25(OH)$_2$D$_3$ are dispersed in a small tablet core (e.g. 3-6 mm in diameter). This core is press-coated with a granulate of CaCO$_3$ (containing the equivalent of 250 mg to 500 mg of calcium).
(b) 5-40 µg (mcg) of 24,25(OH)$_2$D$_3$ are dispersed or dissolved in an organic solvent or aqueous mix and the suspension or solution is spray-coated onto a small tablet core. This coated core is then press-coated with CaCO$_3$ granulate (containing the equivalent of 250 mg to 500 mg of calcium).

EXAMPLE 7

Compressed Tablet with Coated 24,25(OH)$_2$D$_3$

5-40 µg (mcg) of 24,25(OH)$_2$D$_3$ are diluted with an inert filler and microencapsulated and then mixed with a CaCO$_3$ granulate (containing the equivalent of 250 mg to 500 mg of calcium). The mixture is then compressed into a tablet.

EXAMPLE 8

Dry-Filled Capsules

(a) 5-40 µg (mcg) of 24,25(OH)$_2$D$_3$ are adsorbed onto a suitable substrate and placed in a capsule. The remainder of the capsule is filled with a suitable form of CaCO$_3$ (containing the equivalent of 250 mg to 500 mg of calcium).
(b) Microencapsulated amounts of 5-40 µg (mcg) of 24,25(OH)$_2$D$_3$ are placed into capsules and the remainder of the capsules is filled with a suitable form of CaCO$_3$ (containing the equivalent of 250 mg to 500 mg of calcium).
(c) A pellet or tablet containing 5-40 µg (mcg) of 24,25(OH)$_2$D$_3$ is placed in a capsule and the remainder of the capsule is filled with a suitable form of CaCO$_3$ (containing the equivalent of 250 mg to 500 mg of calcium).

EXAMPLE 9

Soft Gelatin Capsules

A non-aqueous slurry containing 5-40 µg (mcg) of 24,25(OH)$_2$D$_3$ and CaCO$_3$ (containing an equivalent of 250 mg to 500 mg of calcium) is filled into a soft gelatin capsule.

EXAMPLE 10

Liquid-Filled Hard Capsules

A non-aqueous dispersion containing 5-40 µg (mcg) of 24,25(OH)$_2$D$_3$ and an amount of CaCO$_3$ equivalent to 250 mg to 500 mg of calcium is filled into a hard gelatin capsule.

EXAMPLE 11

Granules/Powders

(a) A suitably flavoured granulate containing 5-40 µg (mcg) of 24,25(OH)$_2$D$_3$ and CaCO$_3$ (containing an equivalent of 250 mg to 500 mg of calcium) is filled into a sachet. This formulation is suitable for dispersing in a glass of a beverage (e.g. water, milk or fruit juice) for immediate consumption.
(b) A unit dose cup containing 5-40 µg (mcg) of 24,25(OH)$_2$D$_3$ and CaCO$_3$ (containing the equivalent of 250 mg to 500 mg of calcium) in granulate/powder form comprising also flavours, thickeners and possibly other excipients. The product is suitable for reconstitution with water to be shaken and drunk as a suspension.

## Claims

### Claims for the following Contracting States : AT, BE, CH, DE, FR, GB, IT, LI, LU, NL, SE

1. A pharmaceutical composition for the alleviation or prevention of osteoporosis in a human patient, comprising a combination of a dosage unit of from 10 to 40 $\mu$g (mcg) of 24,25(OH)$_2$D$_3$ and from 250 to 750 mg of calcium in the form of a conventional calcium supplement, together with a pharmaceutically acceptable carrier or excipient.

2. A composition according to claim 1 comprising from 10 to 20 $\mu$g (mcg) of 24,25(OH$_2$)D$_3$ and from 250 to 500 mg of calcium in the form of a conventional calcium supplement.

3. A composition according to claim 1 or 2 in a form suitable for oral administration.

4. A composition according to any one of claims 1 to 3 wherein the conventional calcium supplement is calcium carbonate.

5. The use in the manufacture of a pharmaceutical composition of 24,25(OH)$_2$ D$_3$ and calcium in the form of a calcium supplement, for the alleviation or prevention of osteoporosis in a human patient.

### Claims for the following Contracting States : GR, ES

1. A method of preparing a pharmaceutical composition for the alleviation or prevention of osteoporosis in a human patient, which method comprises preparing a combination of a dosage unit of from 10 to 40 $\mu$g (mcg) of 24,25(OH)$_2$D$_3$ and from 250 to 750 mg of calcium in the form of a conventional calcium supplement, together with a pharmaceutically acceptable carrier or excipient.

2. A method according to Claim 1 in which the composition comprises from 10 to 20 $\mu$g (mcg) of 24,25(OH$_2$)D$_3$ and from 250 to 500 mg of calcium in the form of a conventional calcium supplement.

3. A method according to Claim 1 or 2 in which the composition is in a form suitable for oral administration.

4. A method according to any one of Claims 1 to 3 wherein the conventional calcium supplement is calcium carbonate.

5. The use in the manufacture of a pharmaceutical composition of 24,25(OH)$_2$D$_3$ and calcium in the form of a calcium supplement, for the alleviation or prevention of osteoporosis in a human patient.

### Patentansprüche

1. Pharmazeutische Zusammensetzung zur Linderung oder Verhinderung von Osteoporose bei einem menschlichen Patienten, die eine Kombination einer Dosiseinheit von 10 bis 40 $\mu$g (mcg) 24,25(OH)$_2$D$_3$ und von 250 bis 750 mg Calcium in Form eines herkömmlichen Calciumzusatzes umfaßt, zusammen mit einem pharmazeutisch annehmbaren Trägerstoff oder Füllstoff.

2. Zusammensetzung nach Anspruch 1, gekennzeichnet durch von 10 bis 20 mg (mcg) 24,25(OH)$_2$D$_3$ und von 250 bis 500 mg Calcium in Form eines herkömmlichen Calciumzusatzes.

3. Zusammensetzung nach Anspruch 1 oder 2, in einer zur oralen Verabreichung geeigneten Form.

4. Zusammensetzung nach einem der Ansprüche 1 bis 3, dadurch gekennzeichnet, daß der herkömmliche Calciumzusatz Calciumcarbonat ist.

5. Verwendung von 24,25(OH)$_2$D$_3$ und Calcium in Form eines Calciumzusatzes bei der Herstellung einer pharmazeutischen Zusammensetzung zur Linderung oder Verhinderung von Osteoporose bei einem menschlichen Patienten.

### Revendications

1. Une composition pharmaceutique pour le soulagement ou la prévention de l'ostéoporose chez l'homme, comprenant une association d'une posologie de 10 à 40 $\mu$g de 24,25(OH)$_2$D$_3$ et de 250 à 750 mg de calcium sous la forme d'un supplément conventionnel de calcium, simultanément à un excipient ou un porteur pharmaceutiquement acceptable.

2. Une composition selon la revendication 1 comprenant de 10 à 20 $\mu$g de 24,25 (OH$_2$)D$_3$ et de 250 à 500 mg de calcium sous la forme d'un supplément conventionnel de calcium.

3. Une composition selon la revendication 1 ou 2 sous une forme appropriée pour l'administration orale.

4. Une composition selon l'une quelconque des revendications 1 à 3 où le supplément conventionnel de calcium est le carbonate de calcium.

5. L'utilisation dans la fabrication d'une composition pharmaceutique de 24,25(OH)$_2$ D$_3$ et de calcium sous la forme d'un supplément de calcium, pour le soulagement ou le prévention de l'ostéoporose chez l'homme.

PERCENT OF CHANGE B.M.C.

Fig.1

PERCENT OF CHANGE B.M.C.

Fig.2

PERCENT OF CHANGE B.M.C.

Calcium + 24,25(OH)$_2$ D$_3$ (40mcg/d)

Fig.3

**PERCENT OF CHANGE B.M.C.**

Ca + Iα(OH) D$_3$(0.5mcg/d) vs. Ca + 24,25(OH)$_2$D$_3$(40mcg/d)

□ MEAN VALUE Ca + Iα(OH) D$_3$
(0.5 mcg/d)

◇ MEAN VALUE Ca + 24,25(OH)$_2$D$_3$
(40 mcg/d)

Fig. 4

Fig.5

Fig.6

TOTAL URINE CALCIUM

Calcium + 24,25 $(OH)_2 D_3$ (40mcg/d)

Fig.7

TOTAL URINE CALCIUM

Ca+Iα(OH)D₃(0.5mcg/d) vs. Ca+24,25(OH)₂D₃(40mcg/d)

Fig.8

Fig. 9

URINARY CALCIUM / CREATININE
Calcium + 24,25 (OH)$_2$D$_3$ (40 mcg/d)

Fig. 10

# URINARY CALCIUM / CREATININE

## $Ca + 1\alpha(OH)D_3 (0.5\,mcg/d)$ vs. $Ca + 24,25(OH)_2D_3 (40\,mcg/d)$

☐   MEAN VALUE $Ca + 1\alpha(OH)D_3$
(0.5 mcg/d)

◇   MEAN VALUE $Ca + 24,25(OH)_2D_3$
(40 mcg/d)

Fig. II